# EUROPEAN PATENT APPLICATION

(11) **EP 1 481 699 A1**
(43) Date of publication of application: **01.12.2004**
(21) Application number: 04102209.6
(22) Date of filing: 19.05.2004
(51) Int. Cl.: A61M 1/10

(54) **Passive non-contacting smart bearing suspension for turbo blood-pumps**

(30) Priority: 28.05.2003 US 473587 P; 14.05.2004 US 845183
(71) Applicant: Goldowsky, Michael P., Valhalla, NY 10595 (US)
(72) Inventor: Goldowsky, Michael P., Valhalla, NY 10595 (US)
(74) Representative: Weydert, Robert

(57) **Abstract**

The ultimate in suplicity and reliability, an axial-flow blood pump (18) is supported by an all passive contact-free hybrid bearing suspension that can measure differential pump pressure. The preferred suspension consists of a radial hydrodynamic journal bearing with axial magnetic thrust bearings (4,4'). The thrust bearing consists of repulsion magnet pairs (7) positioned at each end of the rotor. This holds bidirectional loads with a stable restoring force (negative axial stiffness). Rotor axial position shifts with load and is monitored with a position sensor (16) to inherently provide pump differential pressure (which may be used to physiologically control pump flow rate). The hydrodynamic radial and "smart" axial magnet thrust bearings use large gaps (5,5') to eliminate hemolysis and are actively washed out under pressure with fresh blood to eliminate thrombosis.

## Description

### Field of the Invention

This invention relates to axial or centrifugal turbo blood pumps and more particularly to such blood pumps whose rotor is passively suspended, for measuring left ventricular assist devices (LVAD) differential pressure.

### Claim of Priority

Applicant claims priority based upon a U.S. provisional application filed May 28, 2003 under SN 60/473,587, inventor Michael Goldowsky.

### Background of the Invention

First generation blood pumps utilized and still utilize flexible pumping ventricles in contact with blood. They have no blood immersed bearings and not prone to thrombosis. An advantage is their inherent pulsating flow. For one disadvantage they are too large for competitive use as LVADs (Left Ventricular Assist Devices). Newer second generation turbo pumps have a high rpm impeller like the Jarvik 2000 and Micromed axial flow pumps. They are much smaller but have contacting bearings that suspend the rigid motor. Most second generation pumps are the larger centrifugal type. In axial and centrifugal turbo pumps, bearing contact results in undesirable clot formation either inside or around the periphery of the bearings. Such pumps are not suitable for long term reliable use. A major improvement in turbo pumps has been the relatively recent improvement of incorporating non-contacting bearings to eliminate the main remaining problem of thrombosis. These are known as third generation turbo pumps. Hydrodynamic blood immersed bearings and magnetic bearings are currently state of the art and are in development. No third generation pump presently has U.S. FDA approval for general use. The Incore 1 (trademark) (Berlin Heart Corp., Berlin, Germany) recently received EU Seal of Approval for marketing in Europe. It has a fully magnetically suspended rotor.

Goldowsky patent No. 6,527,699 titled: "Magnetic Suspension Blood Pump" discloses a similar and smaller third generation axial flow miniature turbo pump, whose rotor is suspended both radially and axially using fringing ring non-contacting magnetic bearings. The bearing is radial passive and unstable axially. An active control system is used to stabilize the bearing axially and absorb rotor axial pressure forces. Measuring rotor axial position by implementing virtually zero power feedback control, allows measuring pump differential pressure (on which physiologic control can be based). This is a "smart" magnetic bearing.

A disadvantage of third generation pumps is the fact that an electronic control system is required to stabilize the magnetic bearing. This requires space which is at a premium in implanted devices, particularly in children and small adults. Pump size is to be minimized and this also reduces infection. Control electronics contribute to unreliability not to mention their additional cost. In the above Goldowsky patent, back-up mechanical axial thrust bearings or pins are provided should the control system fail. These are not needed in the instant invention because there is no bearing electronics to fail. The elimination of an actively controlled rotor with its control system in the instant invention is clearly advantageous and extends the present art. Consequently, the primary purpose of the instant invention, is to devise a passive rotor suspension for use in axial as well as centrifugal turbo blood pumps and to provide inherent capability to measure LVAD differential pressure. This shall be called a fourth generation pump.

Some turbo pumps employ radial hydrodynamic journal bearings that eliminate contact. An example is the Cleveland Clinic (Cleveland, OH) centrifugal "Coraid" (trademark). The axial thrust on the impeller is absorbed using the passive magnetic-reluctance force of the motor. There is no teaching in their published or patent literature to monitor impeller axial position as a measure of differential pressure.

Some pumps use a radial load capacity hydrodynamic journal bearing like the above, but with an axial hydrodynamic thrust bearing or a non-desirable contacting thrust bearing to hold axial forces. An example of one with all hydrodynamic bearings is the VentraAssist (trademark) centrifugal, (Ventracor Ltd, Sydney, Australia). It does not measure rotor axial position or differential pressure. Radial magnetic bearing pumps have attempted to employ axial hydrodynamic thrust bearings. Hydrodynamic thrust bearings pose difficult design constraints in order not to damage blood. Their small gap of typically a mil (.001"), characteristically possesses high blood shear which causes hemolysis with potential clotting. Such small gaps are difficult to adequately wash out to eliminate thrombosis. If a hydrodynamic thrust bearing functions, its axial deflection is so small (with typical differential pressures of 0-150mmhg) that deflection cannot be reliably measured to accurately determine differential pressure. Also, the stiffness properties of the bearing are dependent on blood viscosity so its calibration is not constant and changes. These disadvantages are overcome in the instant invention using a passive magnetic thrust bearing that has substantial axial deflection that can be easily and accurately measured independent of blood properties.

Full, non-contacting magnetic suspensions have been a successful approach in both axial and centrifugal pumps. For example, the University of Utah (Salt Lake City, USA) HeartQuest (trademark) centrifugal pump, employs passive radial magnets to support the impeller. However, the impeller is unstable axially so active electronic axial control is used. They do not teach monitoring rotor axial position to determine differential pressure. An example of a successful axial flow pump is the Incore 1 (trademark) (Berlin Heart Corp., Berlin, Germany). It is similar magnetically to the Goldowsky patent cited above, but is four times larger and does not claim to inherently obtain differential pressure.

Ernshaw's Law (circa 1800's) states that: "A rigid body cannot be totally magnetically suspended passively in all axes". There must be at least one axis of instability and this axis requires active control for stabilization. The above pumps exemplify this. Fully magnetically suspended rotors which have active or passive type magnetic bearings are not the ultimate in simplicity and reliability because a control system is required with undesirable electronics.

### Objects and Summary of the Present Invention

Accordingly, there are three primary objects of the present invention. One is to provide a totally passive, non-contacting rotor suspension (requiring no control electronics). Another is to provide an axial thrust bearing possessing sufficient deflection and having a passive restoring stiffness independent of blood properties to absorb rotor axial forces without contact. Thirdly, is the ability to easily measure rotor axial position to obtain LVAD differential pressure.

Other objects of the present invention are to provide a very small yet high force capability magnetic thrust bearing design that is axis symmetric. Another object of the thrust bearing is to shield its fields from interfering with the motor and to provide a linear restoring force chacteristic. Another object is to provide unusually large radial and axial bearing gaps that possess low hemolysis (red blood cell damage) that can be easily washed out to eliminate thrombosis. It is another object to provide forced pressure washout of the hydrodynamic radial bearing to avoid stasis and clotting therein. Another object is to provide unidirectional high pressure washout of the hydrodynamic bearing to eliminate blood stagnation and regurgitation in the bearing gap and at the bearing exit and inlet.

It is also an important object of this invention to reliably and accurately sense differential pressure by incorporating a "smart thrust bearing" (one that is a transducer as well as a bearing). Differential pressure can be the basis for creating not only pulsating flow to mimic the natural heart but to provide physiologic flow control responsive to exercise level. It is yet another object to accomplish physiologic control in a safe manner by sensing and avoiding adverse suction at the pump inlet based (at least in part) on pump differential pressure.

Another object is to advance the state of the art for turbo pumps designed for long term use (years) by minimizing the generation of micro-emboli. This phenomenon has not yet been addressed in the design of present art turbo pumps. It is also an object to improve the hydraulic efficiency of small axial flow turbo pumps by eliminating leakage of blood past the blade tips. A key object of this invention is to provide the ultimate in LVAD mechanical simplicity with minimal electronics, because simplicity enhances reliability. Simplicity also lowers manufacturing cost, which is desirable to satisfy mass markets.

These and other objects of the present invention are provided in a structure for a turbo blood-pump using passive non-contacting smart bearing suspension. The present invention creates a hybrid-bearing, which is not a full magnetic suspension. Since the instant invention does not employ a purely magnetically suspended rotor, the rotor can be totally passive without violating Ernshaw's Law. The invention suspends the rotor radially using a mechanical hydrodynamic journal bearing; these type bearings are finding application in third generation pumps. The magnetic part of the instant suspension is only axial and a passive magnet is employed in this axis. For high quality of life for the patient, providing pulsating flow (to minimize thrombosis and to increase blood perfusion of organs) as well as physiologic flow rate control (responsive to exercise level) is highly desired. The passive axial thrust bearing stiffness of the instant magnet pairs allows tailored and substantial axial deflection of the rotor for accurate measurement (yet the bearing can absorb shock without contact). Monitoring rotor axial position by having a "smart bearing" is one that allows monitoring LVAD differential pressure on which one may, at least in part, base physiologic control. Use of differential pressure for control is claimed in the Goldowsky patent cited above. That this is a practical way to control turbo pumps is documented in a scholarly paper by Giridharan, et al, entitled Modeling and Control of a Brushless DC Axial Flow Ventricular Assist Device, ASAIO Journal volume 48, No.3, 2002.

### Brief Description of the Drawings

The invention, in accordance with preferred and exemplary embodiments, together with additional objects and advantages, is described in the following detailed description and accompanying drawings in which:
FIG.1 is a longitudinal cross section through a preferred cylindrical axial flow turbo pump. FIG. 1 possesses limited detail, but has the essential elements of a similar axial flow pump described in FIG. 1 of Goldowsky patent No. 6,527,699.
FIGs. 2 and 2A are diagrammatic representations, respectively, an end view and a longitudinal cross section, of a plain axially magnetized magnet, and the magnetic field emanating from the two ends thereof.
FIGs. 3 and 3A show, respectively, an end view and a partial longitudinal cross sectional view, through a permanent magnetic thrust bearing with a flux shielding cup similar to what is shown in FIG. 1, but additionally with an inner fringe.
FIGs. 4 and 4A show, respectively, an end view and a longitudinal sectional view of a thrust bearing using a radial magnetized magnet or magnetic segments, without a flux shielding cup.
FIGs. 5 and 5A show, respectively, an end view and a partial longitudinal sectional view, of a still further alternative thrust bearing with fringing rings, but no flux shielding cup.
FIGs. 6 and 6A show, respectively, a longitudinal view and an end view, of a journal bearing, showing the bearing and an integral screw pump at one end thereof, with the length of the bearing portion being labelled "L".
FIG. 7 is a diagrammatic representation, as a longitudinal cross sectional view, through the journal of a hydrodynamic bearing which is fed with blood to a central groove.
FIG. 8 is a partial longitudinal section through an axial flow pump similar to FIG. 1, but utilizes a contact free hydrostatic thrust bearing that employs an integrated screw pump.
FIG. 8A is a non-sectioned enlarged view of the screw thread shown in FIG. 8.
FIG. 9 shows typical characteristic screw pump flow curves of pressure versus flow, for the screw pump shown in FIG. 8.

### Detailed Description of the Invention

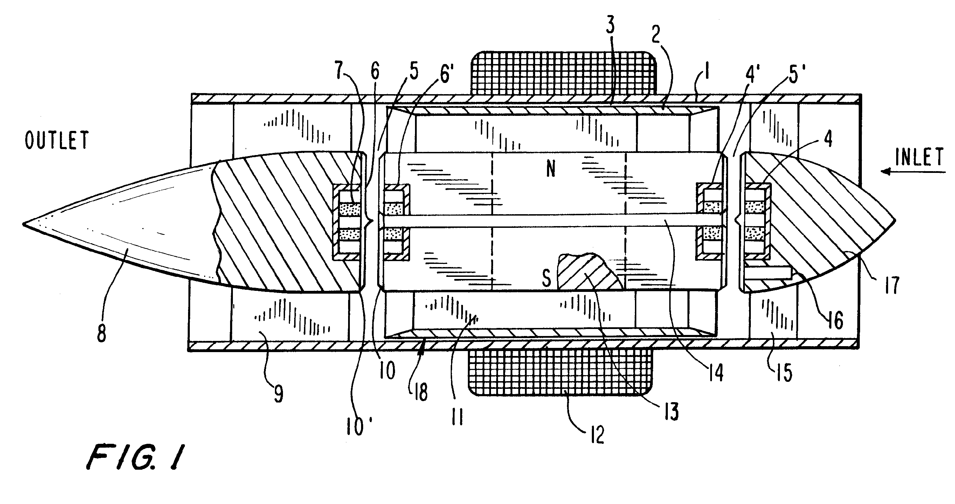

Only the major components of an axial flow turbo pump are shown in FIG. 1 in order to best describe the essential elements of the instant invention. An axial flow pump is illustrated and is not meant to be limiting. The same bearing suspension also applies to use in centrifugal turbo pumps. This is illustrated in the Goldowsky patent cited above.

The pump rotor is generally depicted as item 18. It includes helical impeller blades 11, a round bearing item 2, magnet thrust bearings at each end, items 4 and 4', thin windows 10 and 10', brushless motor armature magnets item 13, and blood flow conduit hole 14 used to wash out the rotor gaps at each end. The concept of employing a central axial hole in the rotor to obtain gap washout flow is contained in Goldowsky U.S. patent No. 6,716,157 "Improved Magnetic Suspension Blood Pump", issued April 6, 2004. The magnet assemblies are hermetically sealed by thin (typically .010 thick) titanium or blood compatible sapphire window items 10. The bearing is separated from pump cylindrical housing 1 by radial blood gap 3, whose combination forms a conventional hydrodynamic bearing. The rotor has large axial blood gaps 5 and 5' at each end. The motor's brushless windings 12 surround the pump housing and are commutated to rotate the rotor.

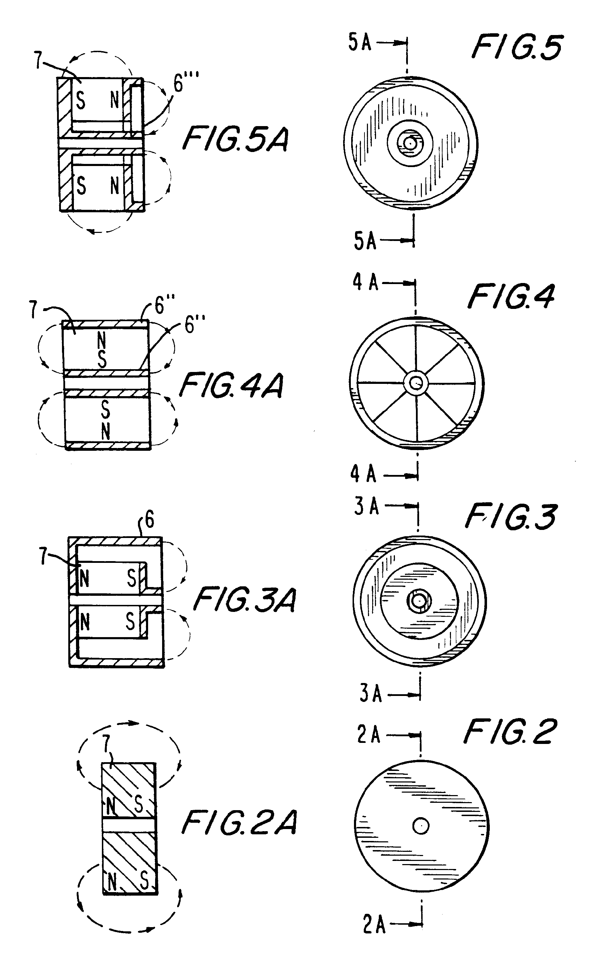

The outlet stator item 8 has a plurality of fixed, flow straightening vanes 9 that are attached to pump housing 1. The inlet stator 17 is fixed to the pump housing by flow inlet vanes 15. Each stator houses a thrust bearing magnet assembly generally designated item 4. A central hole in the magnet is optional, but one is shown as it is preferable that bearing 4 be identical to its co-linear mate 4'. Thrust bearings 4' are preferably located in each end of the rotor as shown to obtain bidirectional force capability. However, only one thrust bearing pair at the LVAD inlet is essential in order to hold differential pump pressure which is directed toward the pump inlet. The symmetrical axial portion of the magnetic fields emanating from each pair of magnets oppose one another making a repelling force. Typical bucking fields are shown dotted in FIGs. 2-5 (the matching bearing mate is not shown). The rotor is repelled in opposite axial directions at each end. This causes the rotor to find a stable central position under axial load. The thrust bearing is axis symmetric to allow rotation and this occurs with very low eddy current losses due to symmetry.

A position sensor 16 uses rotor window item 10 as a target to monitor rotor axial position. The preferred sensor is a miniature ultrasound probe (typically 2mm in diameter) that operates from 5-15 mhz to have low attenuation in blood. It is insensitive to stray magnetic fields emanating from the thrust bearings and motor. The probe tip in contact with blood may have diamond-like coating for blood compatibility. This sensor only requires a flat rigid window surface for its sound-beam to reflect from. In operation, the probe generates a short burst of sound (pulse), and then monitors the time of flight of the return pulse to determine target range. Range is calculated knowing sound velocity in blood which is independent of blood viscosity and composition and gives stable performance. Sound velocity is close to that of pure water and nearly independent of blood type and properties since blood is mostly water. The sensor will not drift and is long term stable; a key requirement for long term blood pump use. This miniature ultrasound sensor is preferred and is claimed in Goldowsky patent No. 6,190,319 titled: Self Calibrating Linear Position Sensor.

Other types of sensors can also be employed such as eddy current, magnetic and capacitance types, but then sensor window and target materials used must be sensor compatible. The position sensor may be conveniently located on or off the pump axis provided it sees the target to measure position of the rotor. An off axis location is depicted in FIG. 1 because this preferred rotor has an on axis blood washout hole, and the hole is an unsuitable target for such a small diameter transducer.

Blood conduit 14 may be a titanium tube with an inside diameter of about 1-2mm. The choice of diameter will control the washout flow rate desired. The conduit may be a titanium tube for blood compatibility. It connects rotor gaps 5 and 5' in series with LVAD differential pressure which (typically 100 mmhg). The resulting flow actively washes out both gaps with frest blood to avoid thrombosis. There is a degree of centrifugal pressure generated in each gap 5 and 5', but they are equal and oppositely directed so their effects cancel. Pump differential pressure is the main driving force that actively washes out these gaps. This is discussed in detail in Goldowsky U.S. patent No. 6,716,157, issued April 6, 2004 entitled: "Improved Magnetic Suspension Blood Pump".

The two essential elements of the instant invention are the passive magnetic thrust bearings and the compact hydrodynamic rotary journal bearing. Thrust bearing details are discussed first as this is new art applied to blood pumps. FIG. 1 shows an axially magnetized cylindrical magnet 7. It is preferably neodymium iron boron or samarium cobalt for very high strength and resistance to the demagnetization load placed on it by its paired opposing magnet. An iron or preferably high saturation vanadium-permendure cylindrical cup 6 surrounds the magnet. The cup retains the magnet's flux and substantially reduces stray fields which could otherwise interfere with the motor armature magnets 18 and vice versa, particularly in a miniature pump where the thrust bearing is desired close to the motor magnets to minimize rotor length. FIG. 3 shows the axial bucking field that emanates (shown dotted) from a similar geometry thrust bearing. In FIG. 1, the cup terminates at its outside diameter as a thin fringing ring item 6 whch is used to focus the magnet flux to a high level (greater than attainable with a pure magnet alone). This flux produces a stronger repulsion force (proportional to gap flux density squared) in a much smaller size than is attainable with a pure magnet alone. FIGs. 2 and 2A show the escaping or stray field emanating from a pure magnet. Its emanating axial field is weak and its axial force is small compared to that when a fringing ring is used. Fringing rings are ideally suited to minimize bearing size which is required in a miniature pump.

FIG. 3 shows a thrust bearing geometry where a second fringing ring is located near to the center. The field emanates from the right end and when a matching bearing faces it, the two fields buck and repel. The closer they approach the higher the repulsion force which acts stably like a mechanical spring (negative stiffness) in the axial direction. However, unlike a mechanical spring, the pair is mutually radial unstable (positive stiffness). The rotor tries to "kick out" at each thrust bearing.

The magnet flux is concentrated higher in the fringe air gap of FIG. 3 than in the fringe air gap of the thrust bearing shown in FIG. 1. This is because the inner fringe has less cross sectional area than the outer, with the same amount of magnet flux, so its flux density is higher. The FIG. 3 bearing is preferred over FIG. 1 for performance but it requires an extra inner fringe.

The hydrodynamic journal bearing is designed very stiffly in the radial direction to easily support the radial instability of the rotor's thrust bearings. Too large a thrust bearing axial stiffness is not desired because axial deflection under load will be too small to easily measure and because its radial instability stiffness becomes undesirably high. For resonance reasons there could be constraints on the journal bearing and thrust bearing stiffness.

These thrust bearing designs allow large gaps for ease of washout as well as large axial deflections. Use of large gaps 5 and 5' allow absorbing the energy of large shock loads without contact even for relatively low axial stiffness. A typical gap may be 0.3-3 mm and axial stiffness is easily tailored to be in the range of 20-200 lb/in.

Another advantage of the iron cup used in bearings of FIGs. 1,3 and 5 is excellent axial force linearity. Constant axial stiffness is desirable for the rotor for dynamic analysis. A linear force displacement characteristic is also desirable for the differential pressure transducer for easy calibration. A pair of plain magnets (a single magnet is shown in FIG.2) is less desirable because it possesses a non-linear or exponential force. It is also much larger for the same stiffness and load capability and it has undesirable stray fields, detrimental in miniature and small pumps.

Whereas, FIGs. 1, 2, 2A, 3 and 3A show a single axial magnetized magnet item 7, the construction of the bearing in FIGs. 4 and 4A utilizes several radial magnetized magnet segments. This is the conventional way to obtain a radial magnetized rare earth magnet (one piece radial magnets are just becoming available). Thin circular iron rings 6" are located at the outside and inside diameters to capture the magnet's flux. The central hole (as in FIG.1 item 14) allows blood to flow therethrough. This configuration is not magnetically efficient because leakage flux passes around the left end as shown and is wasted. This can be compensated for using a longer magnet.

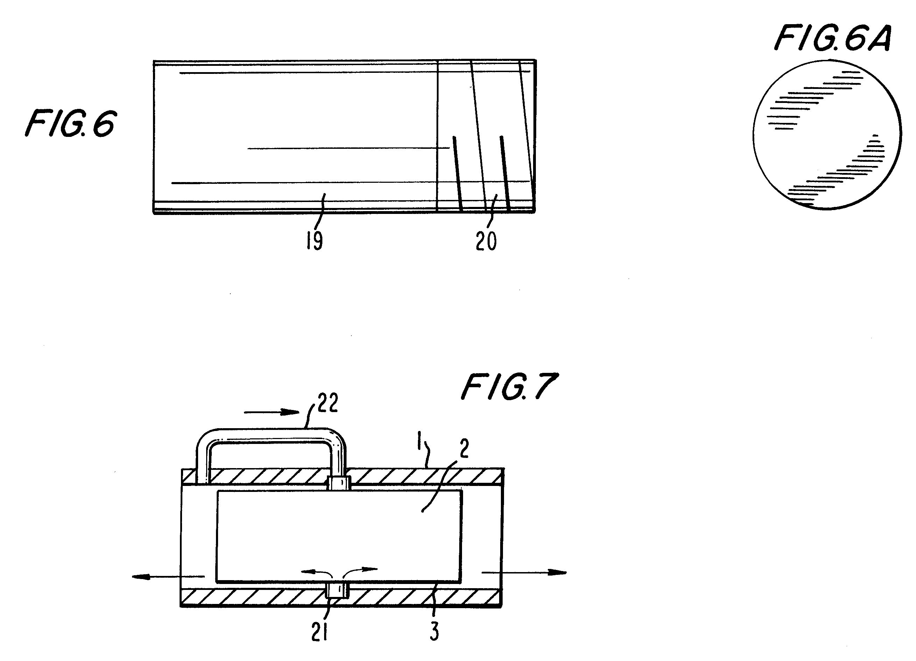

FIG. 5 and 5A are similar in construction to FIG. 1 with an axial magnet but does not have a complete cup to shield the field. Some field is wasted that axially shunts around the outside diameter as shown. An internal finging ring 6' " is added to focus the remaining flux to increase the force. Both outer and inner fringing rings contribute to repulsive force, the inner one generally contributes most of the axial force in FIGs. 3, 3A. 4, 4A, 5 and 5A.

Attention is now directed to the design of the hydrodynamic journal bearing item 2 in FIG. 1. The bearing assembly consists of the thin cylindrical bearing sleeve 2 rotating in round journal 1 axially. It is separated from the journal by radial bearing gap 3 which is blood immersed. The location of the bearing is preferably outside of and surrounds the impeller blades in order to eliminate a blood gap at the O.D. of blades 11 as well as not to occupy the space reserved for the motor magnets in the rotor hub. Blade gaps in conventional axial flow pumps like the Jarvik 2000, must be made small to reduce back leakage past them or pump efficiency suffers. This is most important for the small pumps, especially those designed for children. However, gaps that are too small possess high shear and create hemolysis and can generate micro-emboli which lodge in end organs. The gap has been eliminated in the instant invention. The impeller blade O.D.is bonded to the I.D. of bearing sleeve 2 or is made integral with it. No gap substantially improves pump hydraulic efficiency desirably reducing pump power. The housing bore is straight, rigid and ideally suited to being the journal.

Bearing gap 3 is sized to be sufficiently large at the operating RPM of the pump to possess sub-hemolytic shear stresses. This minimizes blood hemolysis. However, the gap must be sufficiently small to produce the desired load capacity and radial stiffness over the LVAD's rpm operating envelope with variable blood viscosities. A larger gap is desired for low power loss. These constraints have been met in the instant invention. Practical operating gaps may fall in the range up to about 2mm for axial flow pumps typically operating 7,000-20,000 rpm. The design of the hydrodynamic bearing (gap, diameter and length) must be compatible with the impeller and operating rpm. Centrifugal turbo pumps on the other hand usually operate at much lower rpm (1,500-3,000). This necessitates use of a smaller gap.

For a journal bearing to satisfactorily operate in blood it is not sufficient to design just for load capacity. Stagnant areas must be insured against in the gap or blood will clot. To insure against clotting, it has been proven experimentally that supplying fresh blood under pressure to the bearing to wash out the gap can eliminate regions of stagnation and formation of thrombus. FIGs. 6. 6A show a bearing that integrates a pressure generating screw pump at one end item 20 with a bearing portion item 19 of length L. It is similar to those shown in Goldowsky patent Nos. 5,924,975 (Linear Hydrodynamic Blood Pump) and 6,436,027 (Hydrodynamic Blood Bearing). FIGs. 6, 6A are the preferred design of the instant bearing. Only a short pump section 20 is needed particularly in axial flow turbo pumps because rpm is so high.

The screw pump consists of a shallow helical screw thread of multiple starts. Blood is viscosity pumped along the thread grooves that are designed to generate pressure in excess of the outlet pressure of the LVAD (LVAD pressure typically 120 mmhg). The thread need only be a few mils deep allowing sleeve 2 to be thin to require little space. This creates continuous flow through the bearing continuously washing it out. The screw pump is preferably located near the pump inlet which is at low pressure. The screw pumps blood in one direction into the journal bearing gap and it exits at the bearing gap outlet to mix with LVAD blood. Since LVAD bulk blood is in the same direction as the gap flow, the two merge without regurgitation or back flow. Gap flow toward the LVAD outlet thereby eliminates stagnation regions to insure against thrombus formation at both bearing gap inlet and outlet. The screw pump flow is made sufficiently large so blood does not become heated in the bearing and to insure that a generous safety factor exists to wash out the gap under all operating conditions of the LVAD.

If a pressurizing pump (of some type) is not used to wash out the journal bearing gap one must take into account the phenomenon that a hydrodynamic bearing will try to pump fluid out both ends. If no fluid is supplied, the fluid will stagnate in the bearing, and if blood, it will clot. A fluid groove (or inlet holes) is conventionally used in bearings not designed for blood, and is located at the center of the journal. The groove allows fluid to be passively drawn in. In an automobile engine, it is supplied under pressure by an external oil pump. This is possible to implement in the instant invention. One can use a sufficiently large tube that allows passage of fresh blood from the LVAD bulk flow (preferably at the higher pressure outlet) to the groove in the journal. This tube 22 is shown schematically in FIG. 7. The journal groove item 21 typically subtends 360 degrees to make load capacity symmetrical in angular direction and to wash out all areas. Arrows at the groove bottom depict bearing flow out of the groove toward each end of the bearing where it exists. The tube may alternatively be a hole in the wall of housing 1, but this is not practical for a thin wall, unless the wall is locally thickened. However, use of a central groove is not preferable compared to using an integral pump to supply pressurized-flow at the bearing end. A central groove undesirably reduces bearing load capacity by shortening bearing effective length L. This requires increasing bearing length to compensate. Grooves may also be prone to areas of stagnation that may clot with incipient bearing failure.

An integrated compact pump such as the screw pump located at the end of hydrodynamic bearing 19 in FIG. 6, has been reliably used in blood with insignificant blood damage. This is an ideal solution to wash out the bearing. By placing the pump at one end of the bearing, the length of the cylindrical bearing portion 19, labeled L, is maximized and is not broken up as when the screw is located at the center of the bearing. An end location provides a longer effective bearing length that maximizes load capacity and this allows use of a larger radial gap. A larger gap has reduced blood shear stresses and less hemolysis. Hemolysis in an integrated hydrodynamic bearing/screw pump that was designed demonstrated a level for just the bearing that is 25 times lower (0.2) than is characteristic of turbo pumps (5mg/dl).

A high pressure is needed to wash out the gap from one end so flow can be steady and sufficient. If no pressurizing pump is used to wash out the bearing gap, then one can use LVAD differential pressure alone (gap flow will be toward LVAD inlet) which is only about 100 mmhg. The gap can completely wash out using this relatively low pressure only if the internal hydrodynamic pressure pumping effect of the bearing is designed to be less. This internal pressure can be substantial. It is created by the rotating bearing and must be taken into account. Otherwise, a stagnant region will exist in the bearing and blood will clot causing bearing failure. Use of a pressurizing screw pump with sufficient pressure and flow avoids this, it is non-contacting, and it automatically operates with the bearing.

There is another undesirable phenomenon if only LVAD differential pressure is employed for bearing washout. The gap flow in the bearing is then toward the LVAD inlet, but the bulk LVAD flow is toward the outlet. Where the gap flow enters the journal bearing, fluid eddies will form with regions of stagnation, because the flow must reverse direction. This occurs at both ends of the bearing where blood enters and leaves the gap with the potential to create thrombus or clots and to generate micro-emboli. Alternatively, if blood is supplied to a central groove, gap flow opposes pump flow at the LVAD inlet, which can cause thrombus and micro-emboli.

Therefore, pump-less washout has hemodynamic reliability concerns. A unidirectional screw pump that directs bearing gap flow toward the LVAD outlet is superior. It eliminates this problem and will provide superior long term hemodynamic reliability.

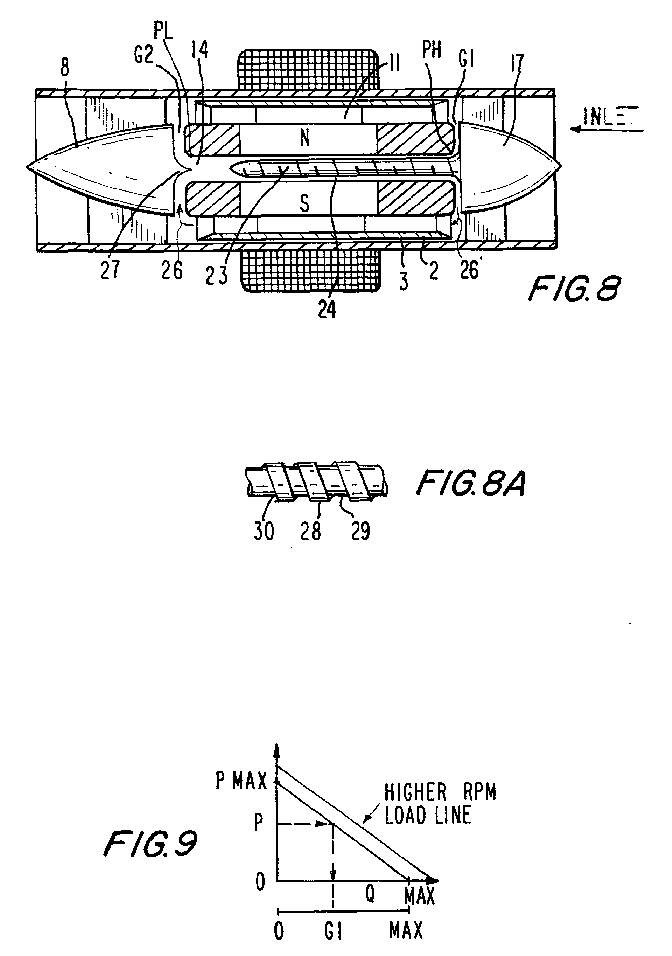

Having thus described the preferred invention, an alternate thrust bearing without magnets, is shown in FIG. 8. This is a hydro-static thrust bearing designed to hold axial load in one direction that is imposed by pump differential pressure. It has the desired stable negative axial stiffness possessing deflections that are much larger than in hydrodynamic thrust bearings. Its axial deflection under LVAD pressure loads is accurately measured using the preferred ultrasonic sensor discussed. A journal bearing with a hydrostatic thrust bearing designed to deliver cooling oil or water to electronic chips is disclosed in Goldowsky patent No. 5,713,670 titled: Self Pressurizing Journal Bearing Assembly. It is not tailored to work in blood but is similar.

The inlet stator 17 (or alternatively outlet stator) uses an attached stationary helical screw 23 that has a single or multiple start thread. It is provided enlarged in FIG. 8A for clarity. Thread lands 28 may be long or short. Thread groove 29 is typically flat-bottomed and corner radii 30 are employed for good washout. The screw is small enough in diameter not to appreciably take space from the motor armature magnets which can be made longer to compensate. Alternately, cylindrical passage 14 may have an internal helical screw thread; in this case a round pin is employed. Alternately, the screw shaft may be supported at both ends. The screw is located on the rotor center line with a radial gap 24 that never contacts by virtue of the stiffness of hydrodynamic bearing 2 whose gap is 3. Rotation of the rotor forces blood to flow and its pressure to increase along the screw helical groove (toward LVAD inlet). Blood enters the screw from large gap G2 in communication with LVAD bulk flow. There is little pressure drop through G2. So PL is nearly the same as rotor outlet pressure. This series flow washes out gap G2 as shown by arrows 26 and 26'. A pointed flow director 27, with a blend radius removes turbulence and allows the flow to smoothly enter central conduit 14. It also is a stop to insure that gap G2 is large at initial start up of the pump so the rotor initially lifts off axially toward LVAD inlet.

The tip of the screw may be an off-center point to divert flow non-symmetrically without a stagnation point, as may be the design of item 27. PL is increased by screw pump 23 to higher value PH at the screw outlet which enters gap G1. Gap G1 is much smaller than gap G2, thereby becoming the primary flow resistance on the screw pump. It is large enough to be subhemolytic. G1 forms a hydrostatic thrust bearing by virtue of the high pressure therein. Its thrust bearing area is equal to the end face area of the rotor hub (which is preferably the same at both ends as shown).

Gap G1 discharges blood 26' to LVAD inlet pressure which is low. The (average pressure acting on rotor face G1 minus pressure PL on rotor face G2) multiplied by rotor hub area, is the net force of the thrust bearing acting toward the LVAD outlet. This balances the entire differential pressure force on the rotor (including its impeller blades). This unique combination or system of a screw pump and thrust bearing, automatically adjusts rotor gap G1 using hydraulic feedback until the thrust bearing force equals the externally applied rotor force. This automatic adjusting of rotor axial position is measured with a position sensor from which differential pressure is calculated by dividing by the effective area of the rotor.

A feedback system exists to drive rotor error position to zero because of the negative slope (pressure P versus flow Q) being inherent in the characteristic curves of screw pump 23. Schematic screw pump curves are shown in FIG. 9. The two "curves" are linear with the upper one being at higher rpm. These lines are parallel and linearly spaced apart as a function of rpm. When screw pump flow rate Q is a maximum value (Q max), zero pressure rise is generated and when Q is zero, maximum pressure rise is generated. The amount of flow Q at a given pressure P is shown dotted which gives the typical operating point; and so operating gap G1 results, it being shown on the lower horizontal scale. When G1 is zero, no flow can occur and when G1 is some maximum value, pressure P drops to zero. G2 is always large even at bearing lift-off and has no effect.

Feedback is stable. Note that if G1 becomes smaller for a given load on the rotor, P increases forcing G1 to increase. If G1 increases too much, P will decrease and so does the thrust bearing force. This reduces G1. At equilibrium, G1 reaches a steady state value and no longer changes. This all happens quickly enabling transient differential pressures to be measured.

This pressure transducer provides a means to determine the onset of inlet suction so it can be avoided. A fast-rising spike in differential pressure and its magnitude characterize suction. Avoiding this is important to obtain safe physiologic control with pulsating flow. Constant rpm LVADs have much less of a problem because their flow rates can be conservatively set without providing pulsating flow. The instant pressure transducer allows one to measure the pressure produced by the patient's beating heart (and the heart's state of recovery) independently of the pulsating pressure produced by the LVAD (which can be programmed at a differing frequency). Also, by maintaining systolic and diastolic differential pressures at preset reference values using cyclic rpm changes, one may provide a means for automatic flow rate control responsive to exercise. These methods are claimed in Goldowsky patent No. 6,527,699 for a totally magnetically suspended pump.

A screw pump operates by pumping fluid (blood) along the screw whose gross flow rate is theoretically independent of fluid viscosity. However, forward gross flow is reduced by back-leakage over the threads which depend on blood viscosity (for laminar flow past the threads). Laminar back flow can be made very small using long lands 28 instead of a smaller gap which has undesirable higher shear stress. A long length screw is advantageous to accomplish this (a long summed length of series lands) because space exists for a long screw in this preferred design. One may also elect to use a larger gap 24 with short thread lands to produce orifice flow. Orifice back flow is viscosity independent. Therefore, it is clear thrust bearing calibration can be made insensitive to blood viscosity (which is variable with hematocrit) for reliable long-term use.

The proposed thrust bearing's (force - deflection) characteristic is linear because a linear load line screw pump is employed as the pressure source (P versus Q is linear). However, rpm must be used to know which characteristic pump curve is in effect. This gives the final piece of real time information needed to calibrate rotor displacement. If a centrifugal type pump is used as the pressure source instead of a screw, then a plain central hole 14 is employed like in FIG. 1. The P versus Q curve is non-linear for centrifugal pumps and pressure rise depends on the square of rpm. A centrifugal type pump is usable (if employed on its negative slope) since its characteristic curves are insensitive to blood viscosity. A centrifugal pump is integrated in the instant invention by providing a plurality of small blades on the rotor in gap G1. This is shown in FIG. 16 of the Goldowsky patent No. 6,527,699 titled: Magnetic Blood Pump, to wash out the bearing gaps of the rotor.

In the event a large axial impact load is applied before the thrust bearing reacts, or if a steady load should exceed the thrust bearing's capability, the rotor will contact the end stators if even momentarily. These are typically titanium surfaces that can be coated with blood compatible diamond like carbon to provide low friction and high hardness to avoid damage. This, or a similar coating, should be employed for safety in the preferred embodiment of FIG. 1.

Whereas, what is described herein are considered to be preferred and exemplary embodiments of the present invention, other modifications shall be apparent to those skilled in the art from the teachings herein, and it is therefore desired to be secured in the appended claims all modifications that fall within the spirit and scope of the invention:

## Claims

1. An implantable blood pump comprising:
(a) a pump rotor having an axis;
(b) a housing for said pump rotor, said housing defining an inlet and an outlet;
(c) a gap defined by said pump rotor, between said pump rotor and said housing;
(d) a magnetic axial thrust bearing acting across said gap to counteract forces on said pump rotor; and
(e) said pump rotor being rotatable, non-contacting and suspended axially within said housing, by said magnets of said thrust bearing, said gap having fresh blood continuously provided thereto for washing to prevent thrombus therein.

2. The blood pump according to claim 1, wherein said blood pump further comprises a hydrodynamic bearing for suspending said pump rotor passively and radially.

3. The blood pump according to claim 1, wherein said magnetic thrust bearing acts as a permanent repulsion magnet.

4. The blood pump according to claim 3, wherein said pump rotor has inlet and outlet ends, a stator proximate each end, and individual repulsion permanent magnets at each end thereof to repel said stators.

5. The blood pump according to claim 1, wherein a rotor position sensor is provided to determine pump rotor differential pressure.

6. The blood pump according to claim 1, wherein said pump rotor defines a conduit to wash out said gap under pressure.

7. The blood pump according to claim 6, wherein said pump rotor defines an axial hole to wash out said gap.

8. The blood pump according to claim 1, wherein said pump rotor has a single axially magnetized permanent magnet, whose field acts at each end of the rotor.

9. The blood pump according to claim 1, wherein said magnet includes a generally centrally positioned rotor magnet disc, with respect to said pump rotor axis, with stator axial magnet discs on either side of, and separated from, said centrally positioned disc, said separations functioning as additional blood flow gaps.

10. The blood pump according to claim 1, wherein said blood pump also includes force generating coils for axially stabilizing said pump rotor.

11. The blood pump according to claim 10 wherein said pump rotor has inlet and outlet ends, and a stator proximate each of said ends, said coils being located in each of said stators.

12. The blood pump according to claim 11, wherein position sensor feedback means is provided to measure pump rotor differential pressure, to actively control current of said coils.

13. A passive rotor suspension structure for an implantable blood pump comprising:
(a) a non-contacting rotor;
(b) a hydrostatic axial thrust bearing to hold external force on said rotor;
(c) said thrust bearing defining a gap; and
(d) a pump to supply pressurized blood to said thrust bearing for continuously washing said gap with fresh blood to eliminate thrombus.

14. A passive rotor suspension structure according to claim 13, wherein a pair of oppositely directed axial thrust bearings are provided to enable a bidirectional force capability.

15. A passive rotor suspension structure according to claim 13, wherein a screw pump is provided fluidically in series with said gap to provide automatic feedback control of the axial position of said rotor, and a sensor for determining external forces on said rotor is included.

16. A passive rotor suspension structure according to claim 13, wherein a centrifugal pump is provided fluidically in series with said gap to provide automatic feedback control of the axial position of said rotor, and a sensor for determining external forces on said rotor is included.

17. A passive rotor suspension structure according to claim 13, wherein said pump further includes a magnetic bearing for radially suspending said rotor.

18. A passive rotor suspension structure according to claim 13, wherein said pump further includes a hydrodynamic bearing for radially suspending said rotor.
